# EUROPEAN PATENT SPECIFICATION

(11) **EP 2 849 104 B1**
(45) Date of publication and mention of the grant of the patent: **27.10.2021**
(21) Application number: 14183967.0
(22) Date of filing: 08.09.2014
(51) Int. Cl.: G16H 10/60, G16H 15/00, G16H 50/50, G16H 50/70

(54) **Medical care information display control apparatus, method, and medium with medical care information display control program recorded thereon**
Vorrichtung zur Steuerung der Anzeige medizinischer Pflegeinformationen, Verfahren und Medium mit darauf aufgezeichnetem Programm zur Steuerung der Anzeige medizinischer Pflegeinformationen
Commande d'affichage d'informations de soins médicaux, appareil, procédé et support d'information de soin médical sur lequel est enregistré un programme de commande d'affichage

(30) Priority: 13.09.2013 JP 2013190060
(43) Date of publication of application: 18.03.2015
(73) Proprietor: FUJIFILM Corporation, Tokyo 106-8620 (JP)
(72) Inventor: Hoshino, Takashi, Kanagawa-ken 258-8538 (JP)
(74) Representative: Klunker IP Patentanwälte PartG mbB

(56) References cited:
- US-A1- 2010 057 646
- US-A1- 2012 102 304
- ALEXANDER RIND: "Interactive Information Visualization to Explore and Query Electronic Health Records", FOUNDATIONS AND TRENDS IN HUMAN-COMPUTER INTERACTION, vol. 5, no. 3, 1 January 2013 (2013-01-01) , pages 207-298, XP055201005, ISSN: 1551-3955, DOI: 10.1561/1100000039
- None

## Description

### BACKGROUND OF THE INVENTION

### Field of the Invention

The present invention relates to a medical care information display control apparatus, method, and program for displaying medical care data of a plurality of medical care items.

### Description of the Related Art

Recently, when a diagnosis is performed by a doctor, processing in which various types of medical care data obtained for patients are displayed on a display device is performed, as reference information for diagnosing disease name and determining treatment policy.

But, there are a large number of medical care items of medical care data obtained for patients and, in particular, if the patients have a plurality of diseases, the number of medical care items is further increased. Therefore, if all medical care data of the large number of medical care items are displayed, it is very difficult to observe the data and an abnormal sign, if any, in the medical care data may possibly be overlooked.

In contrast, Japanese Unexamined Patent Publication No. 2004-078310 discloses a method in which a user specification of a medical care item of interest is received and only the medical care data of the specified medical care item is displayed. But this method has a problem that the user needs to perform an operation to specify a medical care item of interest one by one, thereby requiring a lot of time and effort.

Japanese Unexamined Patent Publication No. 2003-099531 discloses a method in which a display item set that relates a plurality of medical care items to be used in diagnosis of disease is provided in advance with respect to each disease and, if a patient is suspected to have a specific disease, a medical care item to be displayed on a display screen is selected using the display item set corresponding to the disease.

US 2010/057646 A1 discloses a system displaying medical care information, the system having an information obtaining unit that obtains information of medical care data of a patient corresponding to each of plural medical items, a display item set generation unit for extracting medical care data by using medical care data of a first patient and information of the registration date and time of the medical care data. There is generated a display item set composed of the medical care item corresponding to the extracted medical care data. Further, there is provided a display control unit.

Alexander Rind: "Interactive Information Visualization to Explore and Query Electronic Health Records", Foundations and Trends in Computer-Human Interaction, vol. 5, nr. 3, 1st January 2013 (2013-01-01), pages 207-298, XP0055201005, ISSN: 1551-3955 discloses a visualization system for exploring and querying electronic health records.

### SUMMARY OF THE INVENTION

In the meantime, the generation of each display item set poses a problem that it requires a lot of time and effort, as the current situation is that each of a plurality of medical care items to be included in each display item set is relied upon one-by-one manual selection by the doctors and the like based on the knowledge of medical diagnosis.

In view of the circumstances described above, it is an object of the present invention to provide a medical care information display control apparatus, method, and program capable of generating a display item set easily and efficiently with respect to each disease, without requiring the operation that requires time and effort for selecting a plurality of medical care items to be included in a display item set one by one.

A medical care information display control apparatus of the present invention includes the features of claim 1 or claim 2.

Here, the second patient may be the same as or different from the first patient.

The "medical care data" refers to a wide variety of medical care data obtained for diagnosis and treatment of a patient and examples may include test information, such as sample test information, imaging test information, and the like, treatment information, such as medication information and the like, diagnostic information, such as an electronic medical record or discharge summary in which diagnostic information, such as the diagnosis, symptom, surgical treatment of the display target patient is recorded by the doctor, and biological information, such as the body temperature, blood pressure, respiratory rate, and the like of the display target patient.

The "registration date and time of the medical care data" may be expressed in any unit, including month, week, day, and time, and may be any predetermined time arbitrarily set with respect to each medical care item, such as the time when the medical care data were inputted (obtained) first or the time when the medical data were updated last. For example, if it is an imaging test, the registration date and time may be the time when the image was obtained. If it is a sample test, the registration date and time may be the time when the sample test was conducted, if it is an electronic medical record, the registration date and time may be the time when the electronic medical record was created or the time when the record was updated last, if it is medication information, the registration date and time may be the time when the drug was prescribed, and if it is biological information, the registration date and time may be the time when the biological information was measured.

The "disease duration from the start to the end of disease" refers to a period in which medical care data tied up to the disease is present and the period may be identified based on the retained information of the start and end times of the disease inputted by the doctor or the like. More specifically, the period of the start to the end times of the disease may be set as the disease duration and if information of only the start time is available, the start to the present times may be set as the disease duration.

The "displays the medical care data corresponding to the medical care item of the display item set" basically refers to display only the medical care data corresponding to the medical care item of the display item set and not to display medical care data other than the medical care item of the display item set, but, if another display item set generated in advance is selected to be displayed, it refers to that the medical care data corresponding to the medical care item of the selected another display item set is also displayed.

In the medical care information display control apparatus of the present invention, the information obtaining unit obtains medical care data, registration date and time of the medical care data, and information of disease duration of each disease with respect to the first patient, but the information obtaining unit may be a unit that obtains, with respect to the second patient, at least medical care data.

In the medical care information display control apparatus of the present invention, if a plurality of diseases is diagnosed in the first patient, the non-overlapping period identification unit may be a unit that sets any one of the plurality of diseases as the target disease and performs the identification of the non-overlapping period of the target disease or sets each of the plurality of diseases as the target disease in order and repeats the identification of the non-overlapping period of the target disease. Of course, if only one disease is diagnosed in the first patient, the non-overlapping period identification unit performs, with the one disease as the target disease, acquisition of the non-overlapping period of the target disease.

If medical care data of the first patient is additionally obtained by the information obtaining unit after the display item set is generated and a medical care item is newly added to the medical care item corresponding to the medical care data whose registration date and time is included in the non-overlapping period, the display item set generation unit may be a unit that updates the generated display item set by further including the added medical care item.

Here, to perform the highlighted display of a specific medical care item refers to that the medical care data of the medical care item are displayed in a form more visually recognizable than the other medical care data. For example, the medical care data of the medical care item are color-displayed while the other medical care data are displayed in a pale gray so as to be less noticeable, or the medical care data of the medical care item are displayed by a thicker line than that of the other medical care data. Further, to perform the detailed display of a specific medical care item refers to that hidden information with respect to the medical care data of the medical care item is further displayed.

When counting the number of times of user input specifying the highlighted display or the detailed display, the specification of highlighted display and the specification of detailed display may be counted jointly or separately.

In the medical care information display control apparatus of the present invention, the non-overlapping period identification unit is a unit that identifies the non-overlapping period of the target disease automatically or based on a manual user input. For example, if a plurality of diseases is diagnosed in the first patient, the non-overlapping period identification unit may be a unit that displays information of the disease duration of each disease of the first patient on the display screen, receives a user input specifying a period based on the displayed information, and identifies the specified period as the non-overlapping period of the target disease.

Further, if a plurality of diseases is diagnosed in the first patient, the non-overlapping period identification unit may be a unit that obtains a non-overlapping period of each disease using the information of the disease duration of each disease of the first patient, selectably displays the information of the non-overlapping period obtained for each disease on the display screen, receives a user input selecting any one of the displayed non-overlapping periods, and identifies the selected non-overlapping period as the non-overlapping period.

A medical care information display control method according to the present invention is a method defined by claim 8 or claim 9.

A medical care information display control program according to the present invention is defined by claim 10.

According to the medical care information display control apparatus of the present invention, using information of disease duration of each disease of a first patient, a non-overlapping period of disease duration of a target disease, which is one of the diseases of the first patient, that does no overlap with disease duration of the other diseases is identified, then using medical care data of the first patient and information of the registration date and time of the medical care data, medical care data whose registration date and time is included in the non-overlapping period of the target disease is extracted from the medical care data of the first patient and a display item set composed of the medical care item corresponding to the extracted medical care data is generated. That is, assuming a target disease, medical care items having medical care data are automatically identified as medical care items to be included in the display item set and a medical item set composed of the identified medical care items is generated. This allows a display item set with respect each disease to be generated easily and efficiently without requiring the time and effort consuming operation of selecting, one-by-one, the medical care items to be included in the display item set. The generated display item set may be used widely such as the case in which medical care data are displayed with respect to a second patient suspicious of the target disease, and the like.

### BRIEF DESCRIPTION OF THE DRAWINGS

Figure 1 is a block diagram of a medical information system that uses one embodiment of a medical care information display control apparatus of the present invention, illustrating a configuration thereof.
Figure 2 shows an example of medical care information and disease duration with respect to each disease obtained by the information obtaining unit.
Figure 3 shows an example setting screen for receiving a disease to be set as a target of a display item set and a period.
Figure 4 shows an example setting screen for receiving a disease to be set as a target of a display item set and a period.
Figure 5 shows an example of medical care information obtained by the information obtaining unit and data acquisition information with respect to each disease.
Figure 6 shows an example medical care information display screen displayed by the display control unit.
Figure 7 show an example setting screen for receiving a disease name to be added and setting of a display item set.

### DESCRIPTION OF THE PREFERRED EMBODIMENTS

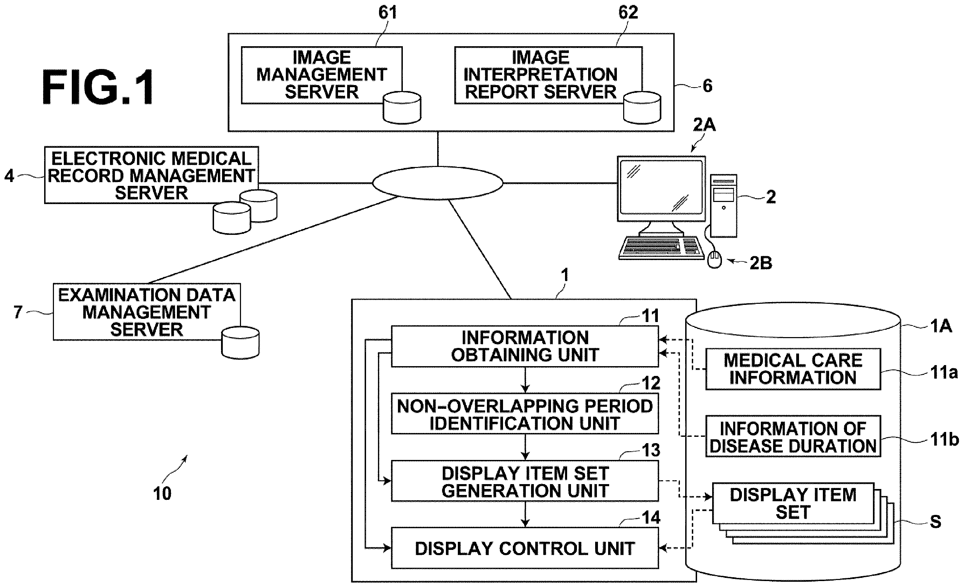

Hereinafter, a medical information system to which a medical care information display control apparatus according to an embodiment of the present invention is applied will be described. Figure 1 is schematic configuration diagram of the medical information system 10 and a functional block diagram of the medical care information display control apparatus 1.

As illustrated in Figure 1, a medical information system 10 includes a medical care information display control apparatus 1, a diagnosis and treatment department terminal 2, an electronic medical record management server 4, an image diagnosis system 6, and an examination data management server 7 which are communicatively connected via a network.

The electronic medical record management server 4 is a computer provided with an electronic medical record database which stores electronic medical records, and includes software for retrieving medical care information, such as an image related to each electronic medical record, test results, or the like, and performing transmission/reception of retrieval results in response to a request from the diagnosis and treatment department terminal 2 or the like, in addition to an operating system and database management software. The electronic medical record management server 4 is connected to the medical care information display control apparatus 1, the diagnosis and treatment department terminal 2, the examination data management server 7, the image diagnosis system 6, and the like via a network so as to be able to obtain each medical care information related to electronic medical records.

The examination data management server 7 is a computer provided with an examination data management database which stores test data, and includes examination data management software in addition to standard software, such as an operating system. Sample test information that includes test date of a sample test performed in the test room according to a test order inputted from each diagnosis and treatment department terminal 2, and test result data is inputted related to the test order and the patient ID from a test room terminal (not shown) provided in each test room and stored in the examination data management database via a network.

The image diagnosis system 6 is a known computer system. Here, it is of a configuration in which a workstation (not shown) for image diagnostic doctors, modalities (not shown), such as CT, MRI, and the like, an image management server 61 provided with an image database which stores image data obtained by the modalities, such as CT, MRI, and the like, and an image interpretation report server 62 provided with an image interpretation report database which stores image interpretation reports, including image interpretation results of the images obtained by the imaging, are communicatively connected via a network. The workstation for image diagnostic doctors includes various types of image analysis software and is configured so as to be able to perform various types of image analysis processing according to the purpose and target of the diagnosis.

The diagnosis and treatment department terminal 2 is a computer used by the doctors of diagnosis and treatment department and the like for viewing medical care information of a patient and inputting a test order, and the like, and includes a display device 2A which is a common display and an input device 2B which includes a keyboard and a mouse. The diagnosis and treatment department terminal 2 is also used for displaying result data of a test performed in each diagnosis and treatment department or medical care information, such as a generated electronic medical record, for reference. The diagnosis and treatment department terminal 2 includes standard software, such as an operation system, and application software for displaying medical care information, such as a generated electronic medical record.

In the present embodiment, when an instructing operation to start display medical care information and a specifying (or inputting) operation of necessary information, such as the patient ID, made by the user, such as a doctor, are received by the input device 2B, the diagnosis and treatment department terminal 2 transmits the various instructions and data corresponding to the various instructions, as required, to the medical care information display control apparatus 1, to be described later. The medical care information display control apparatus 1 receives these various instructions (and corresponding data, as required) and transmits information required to display medical care information of the present embodiment, such as a plurality of medical care information corresponding to the inputted patient ID, and the like, to the diagnosis and treatment department terminal 2. Then, the diagnosis and treatment department terminal 2 receives the display setting information and required information, and displays a medical care information display screen, to be described later, on the display screen of the display device 2A based on the received information.

The medical care information display control apparatus 1 is a computer provided with a medical care information management database 1A. Further, the medical care information display control apparatus 1 includes an operating system and database management software and functions also as a medical care information management server. The medical care information display control apparatus 1 is connected to the electronic medical record management server 4, the diagnosis and treatment department terminal 2, the examination data management server 7, the image management server 61, and the image interpretation report server 62 via a network, and retrieves and obtains medical care information 11a of a patient, such as the electronic medical record of the patient, various test result data, image data, image interpretation report, and the like, from each of the connected servers or the like based on the patient ID, and stores the medical care information in the medical care information management server 1A related to the patient ID. In the medical care information, each medical care information included in the medical care information is stored related to a registration date and time (registration reference date) . The medical care information display control apparatus 1 updates the medical care information to be managed at a fixed time every day. Further, the medical care information display control apparatus 1 receives, as appropriate, medical care information transmitted from each of the servers or the like and updates the medical care information to be managed in response to a request from each of the servers or the like. Still further, the medical care information display control apparatus 1 obtains medical care information from each of the servers or the like and updates the medical care information to be managed as required by the medical care information display control apparatus.

The medical care information management database 1A of the medical care information display control apparatus 1 stores, with respect to each patient ID, information of disease start and end dates (information of disease duration) 11b with respect to each disease diagnosed in the patient. Further, medical care information management database 1A stores a plurality of display item sets S, each set relating, with respect to each disease, a plurality of medical care items to be referred to in diagnosing and treating the disease generated by a display control unit 14, to be described later, and the like.

The medical care information display control apparatus 1 of the present embodiment includes a medical care information display control program according to the present embodiment. By the execution of the medical care information display control program, the medical care information display control apparatus 1 functions as an information obtaining unit 11 that obtains information of medical care data of a patient corresponding to each of a plurality of medical care items, information of registration date and time of the medical care data, and information of disease duration from the start to the end of each disease of the patient correspond to each of a plurality of medical care items, a non-overlapping period identification unit 12 that identifies, using the information of disease duration 11b of each disease of a patient (first patient) obtained by the information obtaining unit 11, a non-overlapping period of disease duration of a target disease, which is one of the diseases of the first patient, that does not overlap with disease duration of the other diseases, a display item set generation unit 13 that extracts, using medical care data of the first patient and information of the registration date and time of the medical care data obtained by the information obtaining unit 11, medical care data whose registration date and time is included in the non-overlapping period of the target disease from the medical care data of the first patient and generates a display item set composed of the medical care item corresponding to the extracted medical care data, and a display control unit 14 that, using information of medical care data of a second patient suspicious of the target disease obtained by the information obtaining unit 11, displays the medical care data of those of the second patient corresponding to the medical care item of the display item set generated by the display item set generation unit. Here, the first patient may be the same as or different from the second patient.

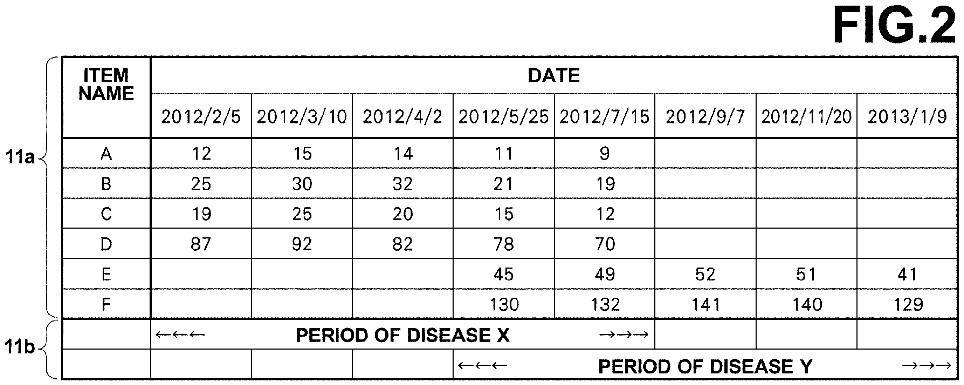

For example, if medical care data 11a of medical care items A to F and disease duration of diseases X and Y shown in Figure 2 are obtained by the information obtaining unit 11 from the medical care information management database 1A with January 10, 2013 as the current date, the non-overlapping period identification unit 12 identify, with either one or each of the diseases X, Y as a target disease, a non-overlapping period of the disease duration of the target disease that does not overlap with disease duration of the other diseases. Then, the display item set generation unit 13 extracts medical care data of the patient whose registration date and time (date) is included in the non-overlapping period among medical care data of the patient and generates display item set composed of the medical care item corresponding to the extracted medical care data. The generated display item set is stored in the medical care information management database 1A related to the target disease.

For example, if the disease X is selected as the target disease, a non-overlapping period (period from February 5, 2012 to April 2, 2012) of the disease duration (period from February 5, 2012 to July 15, 2012) of the disease X that does not overlap with the disease duration (period from May 25, 2012 to January 9, 2013) of the disease Y is identified and a display item set with respect to the disease X composed of a plurality of medical care items A to D corresponding to the medical care data whose registration dates and times (dates) are included in the non-overlapping period. If the disease Y is selected as the target disease, a non-overlapping period (period from September 7, 2012 to January 9, 2013) of the disease duration (period from May 25, 2012 to January 9, 2013) of the disease Y that does not overlap with the disease duration (period from February 5, 2012 to July 15, 2012) of the disease X is identified and a display item set with respect to the disease Y composed of a plurality of medical care items E to F corresponding to the medical care data whose registration dates and times (dates) are included in the non-overlapping period. Note that a display item set with respect to a clinical condition in which the diseases X and Y coexist composed of a plurality of medical care items A to F corresponding to medical care data whose registration dates and times (dates) are included in the period (from May 25 to July 15, 2012) in which the disease durations of the diseases X and Y overlap may also be generated.

At this time, the decision as to which one of the diseases X and Y or both are set as the target diseases maybe made automatically by default setting or based on a user input selecting a disease to be targeted.

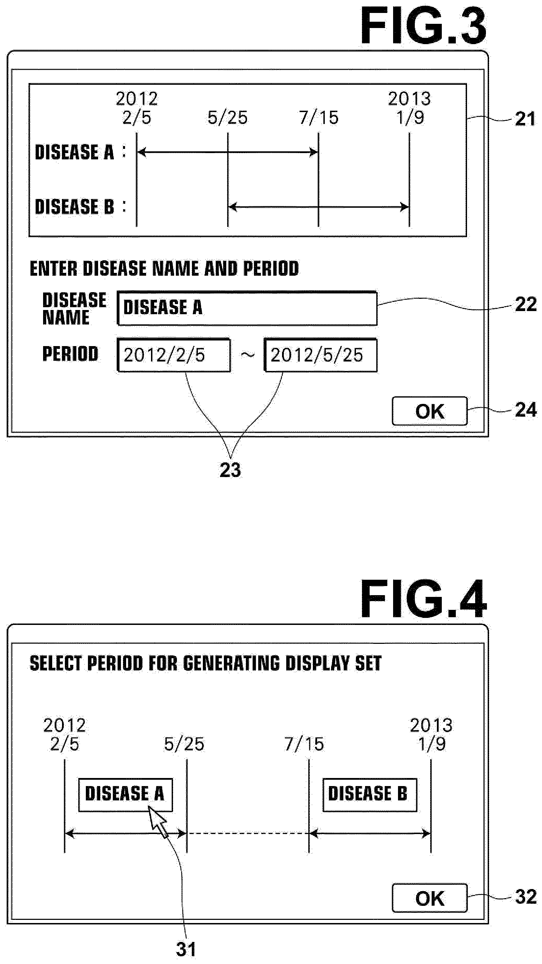

The non-overlapping period identification unit 12 may be a unit that display information of disease duration with respect to each disease of the first patient obtained by the information obtaining unit 11 on a display screen, receive a user input specifying a period based on the displayed information, and set the specified period as the non-overlapping period of the target disease. For example, the non-overlapping period identification unit 12 may display, for example, a setting screen shown in Figure 3, the user may enter a target disease name in the input section 22 and a target period in the period input section 23 by referring to information of disease duration with respect to each disease shown in a display area 21, and the specified period may be set as the non-overlapping period of the target disease when the OK button 24 is pressed by the user. Then, based on the identified non-overlapping period, the display item set generation unit 13 may generate a display item set with respect to the specified disease.

Further, the non-overlapping period identification unit 13 may be a unit that obtains a non-overlapping period with respect to each of a plurality of diseases A, B, then displays, for example, a selection screen shown in Figure 4, receives a user input selecting either one of the periods, and sets the selected period as the non-overlapping period. In Figure 4, the non-overlapping period of each disease is displayed by the disease name button, and when either one of the disease name buttons is selected by the user with a mouse pointer 31 and the OK button 32 is pressed by the user, the period of the selected button is identified as the non-overlapping period of the target disease. Then, based on the identified non-overlapping period, the display item set generation unit 13 may generate a display item set with respect to the specified disease.

If medical care data of the first patient is additionally obtained by the information obtaining unit 11 after the display item set is generated and a medical care item is newly added to the medical care items corresponding to medical care data whose registration dates and times are included in the non-overlapping period to which the display item set is related, the display item set generation unit 13 has a function to automatically update the generated display item set so as to further include the added medical care item.

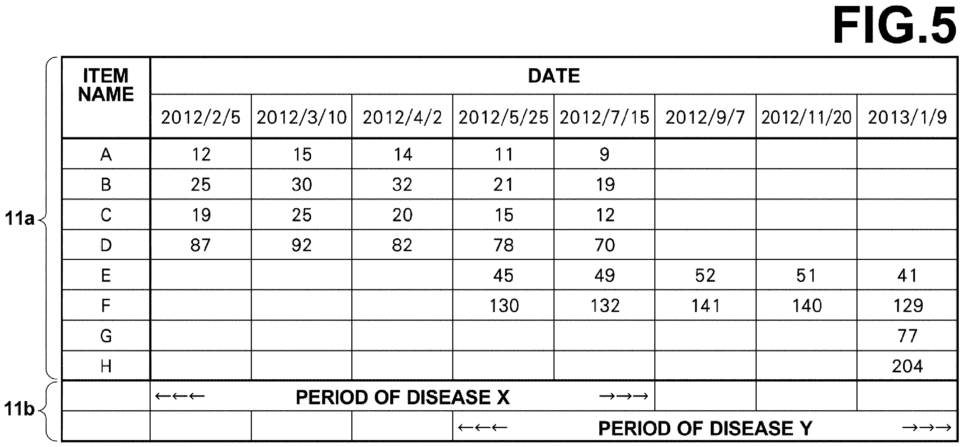

For example, in a case where the non-overlapping period (from September 7, 2012 to January 9, 2013) of the disease Y is identified based on the information of Figure 2 that has been obtained as of January 10, 2013 and a display item set with respect to the disease Y composed of the medical care items E, F corresponding to medical care data whose registration dates and times (dates) are included in the identified non-overlapping period, if, for example, test results G, H performed on January 9, 2013 is added on January 11, 2013, as shown in Figure 5, and the new medical care items G, H are to be added to the medical care items corresponding to medical care data whose registration dates and times (dates) are included in the identified non-overlapping period, the display item set generation unit 13 automatically updates the generated display item set with respect to the disease Y so as to further include the added medical care items G, H.

The medical care data such as the test results are not necessarily registered on the same date as that of the tests performed. Therefore, if an arrangement is adopted in which the presence or absence of an additional medical care item is monitored after the display item set is generated in the manner described above and the display item set is automatically updated, as required, the omission of a medical care item of the display item set may be prevented.

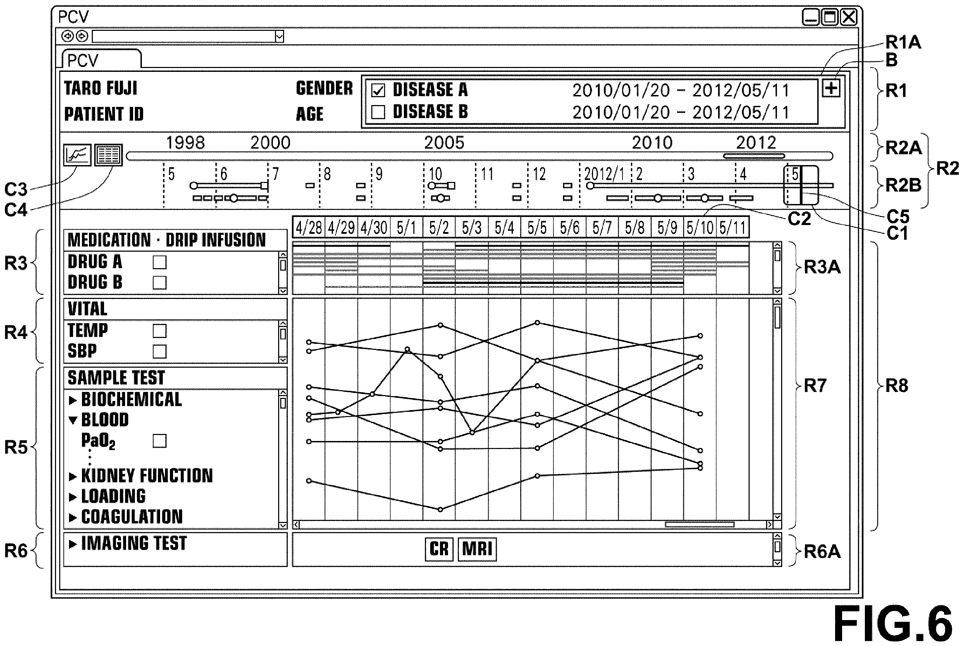

The display control unit 14 generates and displays a medical care information display screen based on the information of medical care data and the like obtained with respect to the second patient who is the medical care information display target. Now, a medical care information display screen display controlled by the display control unit 14 will be described. Figure 6 shows an example display screen which is controlled by the display control unit 14 and displayed on the diagnosis and treatment department terminal 2.

As illustrated in Figure 6, the medical care information display screen includes a basic information display section R1, an examination period display section R2, a medication information display section R3, a medication data display section R3A, a biological information display section R4, a sample test information display section R5, an image information display section R6, an image data display section 6A, and a graph display section R7 for graphically displaying each graphable medical care data set.

The basic information display section R1 is a section in which basic information is displayed, such as the identification information of the second patient (patient ID), name, age, gender, current/past medical histories (diseases), and the like. Further, the basic information display section R1 includes a section R1A in which the current medical history and the past medical history are displayed, and a checkbox is displayed for each disease, as shown in Figure 6. Then, a disease of interest is specified by checking the checkbox. That is, only the medical care data of those of the second patient corresponding to the medical care item of the display item set preset for the disease of interest are displayed on the display screen.

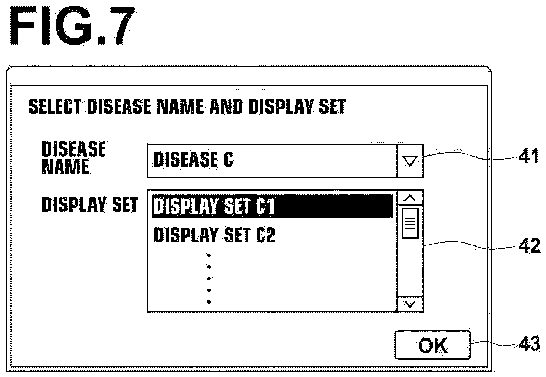

When the disease add button B on the upper light of the current/past medical history section R1A is selected, the display control unit 14 displays, for example, a setting screen shown in Figure 7, then the user selects a disease name to be added (e.g., disease C) in the disease name selection section 41, selects one or more display item sets (e.g., display item set C1) in the display item set selection section 42, and presses the OK button 43, whereby the display of the disease C is added to the current/past medical history display section R1A. At this time, the display item set selection section 42 list displays one or more display item sets stored in the medical care information database 1A related to the disease selected in the disease name selection section 41 according to a predetermined order of priority, and the user may select one or more display item sets from the list. Then, if the checkbox of the disease C is checked in the current/past medical history section R1A, the display is updated so that the medical care data is displayed corresponding to each medical care item of the selected one or more display item sets (e.g., the display item set C1).

The examination period display section R2 is a region representing a time axis. As illustrated in Figure 6, the upper portion R2A of the examination period display section R2 indicates the time axis with the numbers representing Christian years and a bar. Here, the time axis is set to include the period in which medical care data of a patient are present and a predetermined additional period. If the period in which medical care data of a patient are present is shorter than a predetermined period as in a new patient or the like, the time axis is set so as to indicate a predetermined initial period.

The lower portion R2B of the examination period display section R2 indicates the presence of medical care information in the past one year, including the current date, and a rectangular index representing the presence of medical care data is displayed at the position corresponding to each registration date and time of medical care data along the time axis. Further, the examination period display section R2 includes a red line C5 representing the current date displayed at the position corresponding to the current date. Further, a circular index is displayed at the position corresponding to the date of admission and a white square index is displayed at the position corresponding to the date of discharge. Further, the examination period display section R2 includes a rectangular index C1 displayed at the position corresponding to a details display period (period corresponding to the time axis C2 of the medial care data display section R8) whose detailed medical care data are shown in the medical care data display section R8, to be described later, among the periods displayed in the lower portion R2B.

The medication information display section R3 displays a type of drug (medical care item) administered or infused into the display target patient. Further, a medication data display section R3A which shows a period of medication or drip infusion to the patient is provided adjacent to the medication information display section R3, and a period of medication or drip infusion received by the patient is shown by a horizontal bar graph in the medication data display section R3A.

The biological information display section R4 displays a biological information item (medical care item) of the patient. The biological data (medical care data) corresponding to each biological information item in the biological information display section R4 are graphically displayed in the graph display section R7 by a line graph with respect to each biological information item of the patient according to the measurement data (registration date and time) of the biological information. The horizontal axis of the graph display section R7 is the time axis and the vertical axis is the axis that indicates numerical values of each of medical care data, such as the data of biological information, sample test data, and the like.

The sample test information display section R5 displays a sample test item (medical care item) of the patient. The sample test data (medical care data) corresponding to each sample test item displayed in the sample test information display section R5 are graphically displayed in the graph display section R7 by a line graph according to the test date (registration date and time) of each set of test result data. Each medical care item included in sample test information is further classified into a test middle category, such as biochemical test, blood test, kidney function test, loading test, coagulation test, or the like, and each middle category is further classified into a test small category.

The image information display section R6 displays imaging test information of the patient, and title characters are displayed here. Further, an image data display section R6A is provided adjacent to the image information display section R6, and a thumbnail image obtained by imaging the patient with CR or MRI is displayed at the position corresponding to the imaged date (registration date and time) of the image in the image data display section R6A. When the thumbnail image is selected, for example, by clicking or the like, the original image corresponding to the thumbnail image is displayed on a separate display screen with an image interpretation report of the original image.

The medication data display section R3A, the graph display section R7, and the image data display section R6A constitute a medical care data display section R8 in which medical care data of a plurality of medical care items of the patient included in the details display period indicated on the time axis C2 are displayed in time series. In the medical care information display screen, the medical care data displayed in the medical care data display section R8 can be switched between a tabular form and a graph form and if a tabular form display button C4 located on the upper left of the medical care information display screen is selected, the tabular form display is applied and if a graph form display button C3 located on the upper left of the medical care information display screen is selected, the graph form display is applied.

In the medication information display section R3, the biological information display section R4, and the sample test information display section R5, each medical care item is list-displayed and a checkbox is displayed for each test item. Then, medical care data of the medical care item whose checkbox is checked are highlighted in the medical care data display section R8. More specifically, the medical care data of the medical care item whose checkbox is checked are, for example, color-displayed while the medical care data of a medical care item whose checkbox is not checked are displayed in a pale grey so as to be less noticeable.

At this time, the display items set generation unit 13 has a function to count the number of user inputs (e.g., the number of times the checkbox is checked) that specify a highlighted display with respect to each of medical care items of the display item set applied to the medical care information display screen and to generate a display item set composed only of a medical care item of those of the display item set whose counted number of times is greater than or equal to a predetermined threshold value by receiving a user instruction to refine the display item set. At this time, the values of the number of user inputs are stored in the medical care information database 1A related to each medical care item. For example, while a display item set with respect to the disease X composed of the medical care items A to D generated based on the information of Figure 2 is applied to the display, if the results of counting the number of user inputs that specify highlight display with respect to each of the medical care items are "medical care item A: 0; medical care item B: 5; medical care item C: 3; and medical care item D: 5" and a user instruction to refine the display item set with the threshold value 2 is received, a display item set composed of the medical care items B to D is newly generated. At this time, the newly generated display item set is stored in the medical care information management database 1A in place of or in addition to the existing display item set.

Further, the display item set generation unit 13 has a function to count the number of user inputs (e.g., the number of times the checkbox is checked) that specify highlight display with respect to each of medical care items of the display item set applied to the medical care information display screen and to generate a display item set composed only of medical care items of a plurality of medical care items of the display item set whose counted number are less than or equal to a predetermined threshold value arranged in descending order of the counted number of times by receiving a user instruction to refine the display item set. For example, while a display item set with respect to the disease X composed of the medical care items A to D generated based on the information of Figure 2 is applied to the display, if the results of counting the number of user inputs that specify highlight display with respect to each of the medical care items are "medical care item A: 0; medical care item B: 5; medical care item C: 3; and medical care item D: 5" and a user instruction to refine the display item set with the condition of top two items is received, a display item set composed of the medical care items B and D is newly generated. At this time, for medical care items whose numbers of inputs are the same, they are sorted in the order of predetermined priority and refined. The newly generated display item set is stored in the medical care information management database 1A in place of or in addition to the existing display item set.

Then, the display control unit 14 may update the display so that the new display item set generated by the refinement is applied to the display. This allows the user to efficiently refer to medical care data.

As described above, according to the medical information system 10 of the present embodiment, the non-overlapping period identification unit 12 identifies, using information of disease duration with respect to each disease of a first patient obtained by the information obtaining unit 11, a non-overlapping period of disease duration of a target disease, which is one of the diseases of the first patient, that does not overlap with disease durations of the other diseases, the display item set generation unit 13 extracts, using medical care data of the first patient and information of the registration date and time of the medical care data obtained by the information obtaining unit 11, medical care data whose registration date and time is included in the non-overlapping period of the target disease from the medical care data of the first patient and generates a display item set composed of a medical care item corresponding to the extracted medical care data, that is, assuming a target disease, medical care items having medical care data are automatically identified as medical care items to be included in the display item set and a medical item set composed of the identified medical care items is generated. This allows a display item set with respect each disease to be generate easily and efficiently without requiring the time and effort consuming operation of selecting, one-by-one, the medical care items to be included in the display item set. The display item set generated by this may be used widely such as the case in which medical care data are displayed with respect to a second patient suspicious of the target disease, and the like.

Not limited to each embodiment described above, and a part or whole of the components of the medical care information display control apparatus may be configured by one workstation or by more than one workstation, a server, and a storage device connected through a network. Each device is controlled by a program that performs medical care information display described herein installed from a recording medium, such as a CD-ROM or the like. The program may be installed after being downloaded from a storage device of a server connected through a network, such as the Internet.

## Claims

1. A medical care information display control apparatus (1), comprising:
an information obtaining unit (11) adapted to obtain information of medical care data of a patient corresponding to each of a plurality of medical care items, information of registration date and time of the medical care data, and information of disease duration from the start to the end of each disease of the patient;
a non-overlapping period identification unit (12) adapted to identify, using information of disease duration of each disease of a first patient obtained by the information obtaining unit (11), a non-overlapping period of disease duration of a target disease, which is one of the diseases of the first patient, that does not overlap with disease durations of the other diseases;
a display item set generation unit (13) adapted to extract, using medical care data of the first patient and information of the registration date and time of the medical care data obtained by the information obtaining unit (11), medical care data whose registration date and time is included in the non-overlapping period of the target disease from the medical care data of the first patient and generates a display item set composed of the medical care item corresponding to the extracted medical care data; and
a display control unit (14) adapted to display, using information of medical care data of a second patient suspicious of the target disease obtained by the information obtaining unit (11), the medical care data of those of the second patient corresponding to the medical care item of the display item set generated by the display item set generation unit (13) on a display screen.

2. The medical care information display control apparatus as claimed in claim 1, adapted such that, if medical care data of the first patient is additionally obtained by the information obtaining unit (11) after the display item set is generated and a medical care item is newly added to the medical care item corresponding to the medical care data whose registration date and time is included in the non-overlapping period, the display item set generation unit (13) updates the generated display item set by further including the added medical care item.

3. The medical care information display control apparatus as claimed in claim 1 or 2, adapted such that:
the display control unit (14) has a function to perform, in response to a user input to perform a highlighted or a detailed display by specifying one of the medical care items on the display screen in which the medical care data of the second patient corresponding to the medical care item of the display item set is displayed, a highlighted display or a detailed display of medical care data corresponding to the specified medical care item; and
the display item set generation unit (13) has a function to count a number of times of the user input and to generate a display item set composed only of a medical care item of those of the display item set whose counted number of times is greater than or equal to a predetermined threshold value.

4. The medical care information display control apparatus as claimed in claim 1 or 2, adapted such that:
the display control unit (14) has a function to perform, in response to a user input to perform a highlighted or a detailed display by specifying one of the medical care items on the display screen in which the medical care data of the second patient corresponding to the medical care item of the display item set is displayed, a highlighted display or a detailed display of medical care data corresponding to the specified medical care item; and
the display item set generation unit (13) has a function to count a number of times of the user input and to generate a display item set composed only of a number of medical care items of those of the display item set which is less than or equal to a predetermined threshold value in descending order of the counted number of times.

5. The medical care information display control apparatus as claimed in any of claims 1 to 4, adapted such that, if a plurality of diseases is diagnosed in the first patient, the non-overlapping period identification unit (12) sets any one of the plurality of diseases as the target disease and performs the identification of the non-overlapping period of the target disease.

6. The medical care information display control apparatus as claimed in any of claims 1 to 4, adapted such that: if a plurality of diseases is diagnosed in the first patient,
the non-overlapping period identification unit (12) sets each of the plurality of diseases as the target disease in order and repeats the identification of the non-overlapping period of the target disease; and
the display item set generation unit (13) repeats, with respect to each target disease, the generation of the display item set using the information of the non-overlapping period of each target disease.

7. The medical care information display control apparatus as claimed in any of claims 1 to 5, adapted such that: if a plurality of diseases is diagnosed in the first patient, the non-overlapping period identification unit (12) displays information of the disease duration of each disease of the first patient on the display screen, receives a user input specifying a period based on the displayed information, and identifies the specified period as the non-overlapping period of the target disease.

8. The medical care information display control apparatus as claimed in any of claims 1 to 5, adapted such that, if a plurality of diseases is diagnosed in the first patient, the non-overlapping period identification unit (12) obtains a non-overlapping period of each disease using the information of the disease duration of each disease of the first patient, selectably displays the information of the non-overlapping period obtained for each disease on the display screen, receives a user input selecting any one of the displayed non-overlapping periods, and identifies the selected non-overlapping period as the non-overlapping period.

9. A medical care information display control method performed with a medical care information display control apparatus provided with an information obtaining unit (11), a non-overlapping period identification unit (12), a display item set generation unit (13), and a display control unit (14), wherein:
the information obtaining unit (11) obtains information of medical care data of a first patient corresponding to each of a plurality of medical care items, information of a registration date and time of the medical care data, and information of disease duration from the start to the end of each disease of the patient;
the non-overlapping period identification unit (12) identifies, using information of disease duration of each disease of a first patient obtained by the information obtaining unit (11), a non-overlapping period of disease duration of a target disease, which is one of the diseases of the first patient, that does not overlap with disease durations of the other diseases;
the display item set generation unit (13) extracts, using medical care data of the first patient and information of the registration date and time of the medical care data obtained by the information obtaining unit (11), medical care data whose registration date and time is included in the non-overlapping period of the target disease from the medical care data of the first patient and generates a display item set composed of the medical care item corresponding to the extracted medical care data;
the information obtaining unit (11) obtains information of medical care data of a second patient suspicious of the target disease corresponding to each of a plurality of medical care items; and
the display control unit (14) displays, using the information of medical care data of the second patient obtained by the information obtaining unit (11), the medical care data of those of the second patient corresponding to the medical care item of the display item set generated by the display item set generation unit (13) on a display screen.

10. A medium on which is recorded a medical care information display control program for causing a computer to function as:
an information obtaining unit (11) adapted to obtain information of medical care data of a patient corresponding to each of a plurality of medical care items, information of a registration date and time of the medical care data, and information of disease duration from the start to the end of each disease of the patient;
a non-overlapping period identification unit (12) that is adapted to identify, using information of disease duration of each disease of a first patient obtained by the information obtaining unit (11), a non-overlapping period of disease duration of a target disease, which is one of the diseases of the first patient, that does no overlap with disease durations of the other diseases;
a display item set generation unit (13) that is adapted to extract, using medical care data of the first patient and information of the registration date and time of the medical care data obtained by the information obtaining unit (11), medical care data whose registration date and time is included in the non-overlapping period of the target disease from the medical care data of the first patient and generates a display item set composed of the medical care item corresponding to the extracted medical care data; and
a display control unit (14) that is adapted to display, using information of medical care data of a second patient suspicious of the target disease obtained by the information obtaining unit (11), the medical care data of those of the second patient corresponding to the medical care item of the display item set generated by the display item set generation unit (13) on a display screen.

## Patentansprüche

1. Anzeigesteuervorrichtung (1), für medizinische Pflegeinformation, umfassend:
eine Informationsgewinnungseinheit (11), ausgebildet zum Gewinnen von Information medizinischer Pflegedaten eines Patienten, entsprechend jedem von mehreren medizinischen Pflegeposten, Information von Registrierungsdatum und - zeit der medizinischen Pflegedaten, und Information über Krankheitsdauer von dem Start bis zum Ende jeder Krankheit des Patienten;
eine Identifikationseinheit (12) für eine überlappungsfreie Zeitspanne, ausgebildet, um anhand von Information über Krankheitsdauer jeder Krankheit eines ersten Patienten, gewonnen von der Informationsgewinnungseinheit (11), eine überlappungsfreie Zeitspanne für Krankheitsdauer einer Zielkrankheit zu identifizieren, bei der es sich um eine von den Krankheiten des ersten Patienten handelt, die sich nicht mit Krankheitsdauern anderer Krankheiten überlappt;
eine Erzeugungseinheit (13) für eine Anzeigepostenmenge, ausgebildet, um unter Verwendung von medizinischen Pflegedaten des ersten Patienten und Information von Registrierungsdatum und -zeit der medizinischen Pflegedaten, die von der Informationsgewinnungseinheit (11) erhalten wurden, aus den medizinischen Pflegedaten des ersten Patienten medizinische Pflegedaten zu extrahieren, deren Registrierungsdatum und -zeit in der überlappungsfreien Zeitspanne enthalten ist, und eine Anzeigepostenmenge zu erzeugen, zusammengesetzt aus dem medizinischen Pflegeposten entsprechend den extrahierten medizinischen Pflegedaten; und
eine Anzeigesteuereinheit (14), ausgebildet, um unter Verwendung von Information der medizinischen Pflegedaten eines zweiten Patienten, der der von der Informationsgewinnungseinheit (11) erhaltenen Zielkrankheit verdächtig ist, die medizinischen Pflegedaten von denen des zweiten Patienten entsprechend dem medizinischen Pflegeposten der Anzeigepostenmenge, die von der Erzeugungseinheit (13) für Anzeigepostenmengen erzeugt wurde, auf einem Anzeigebildschirm anzuzeigen.

2. Anzeigesteuervorrichtung nach Anspruch 1, derart ausgebildet, dass, wenn die medizinischen Pflegedaten des ersten Patienten zusätzlich von der Informationsgewinnungseinheit (11) erhalten wurden, nachdem die Anzeigepostenmenge erzeugt wurde, und ein medizinischer Pflegeposten neuerlich zu dem medizinschen Pflegeposten entsprechend den medizinischen Pflegedaten hinzugefügt wurde, deren Registrierungsdatum und -zeit in der überlappungsfreien Zeitspanne enthalten ist, die Erzeugungseinheit (13) für Anzeigepostenmengen die erzeugte Anzeigepostenmenge aktualisiert wird, indem weiterhin der hinzugefügte medizinische Pflegeposten einbezogen wird.

3. Anzeigesteuervorrichtung nach Anspruch 1 oder 2, derart ausgebildet, dass
die Anzeigesteuereinheit (14) eine Funktion aufweist, um ansprechend auf eine Benutzereingabe zum Ausführen einer hervorgehobenen oder detaillierten Anzeige durch Spezifizieren eines der medizinischen Pflegeposten auf dem Anzeigebildschirm, auf dem die medizinischen Pflegedaten des zweiten Patienten entsprechend dem medizinischen Pflegeposten der Anzeigepostenmenge angezeigt wird, eine hervorgehobene Anzeige oder eine detaillierte Anzeige der medizinischen Pflegedaten entsprechend dem spezifizierten medizinischen Pflegeposten zu bewerkstelligen; und
die Erzeugungseinheit (13) für die Anzeigepostenmenge eine Funktion hat, eine Häufigkeit der Benutzereingabe zu zählen und eine Anzeigepostenmenge zu erzeugen, die sich nur aus einem medizinischen Pflegeposten jener der Anzeigepostenmenge zusammensetzt, deren gezielte Häufigkeit größer oder gleich einem vorbestimmten Schwellenwert ist.

4. Anzeigesteuervorrichtung nach Anspruch 1 oder 2, derart ausgebildet, dass
die Anzeigesteuereinheit (14) eine Funktion aufweist, um ansprechend auf eine Benutzereingabe zum Ausführen einer hervorgehobenen oder detaillierten Anzeige durch Spezifizieren eines der medizinischen Pflegeposten auf dem Anzeigebildschirm, auf dem die medizinischen Pflegedaten des zweiten Patienten entsprechend dem medizinischen Pflegeposten der Anzeigepostenmenge angezeigt wird, eine hervorgehobene Anzeige oder eine detaillierte Anzeige der medizinischen Pflegedaten entsprechend dem spezifizierten medizinischen Pflegeposten zu bewerkstelligen; und
die Erzeugungseinheit (13) für die Anzeigepostenmenge eine Funktion hat, eine Häufigkeit der Benutzereingaben zu zählen und eine Anzeigepostenmenge zu erzeugen, zusammengesetzt nur aus einer Anzahl von medizinischen Pflegeposten jener der Anzeigepostenmenge, die kleiner oder gleich einem vorbestimmten Schwellenwert ist, und zwar in absteigender Reihenfolge der gezählten Häufigkeit.

5. Anzeigesteuervorrichtung nach einem der Ansprüche 1 bis 4, derart ausgebildet, dass, wenn eine Mehrzahl von Krankheiten bei dem ersten Patienten diagnostiziert ist, die Identifikationseinheit (12) für eine überlappungsfreie Zeitspanne eine der mehreren Krankheiten als die Zielkrankheit einstellt und die Identifikation der überlappungsfreien Zeitspanne für die Zielkrankheit bewerkstelligt.

6. Anzeigesteuervorrichtung nach einem der Ansprüche 1 bis 4, derart ausgebildet, dass, wenn mehrere Krankheiten bei dem ersten Patienten diagnostiziert sind,
die Identifikationseinheit (12) für eine überlappungsfreie Zeitspanne jede der mehreren Krankheiten als eine Zielkrankheit nach Reihenfolge einstellt und die Identifikation der überlappungsfreien Zeitspanne der Zielkrankheit wiederholt; und
die Erzeugungseinheit (13) für die Anzeigepostenmenge in Bezug auf jede Zielkrankheit das Erzeugen der Anzeigepostenmenge unter Verwendung der Information über die überlappungsfreie Zeitspanne jeder Zielkrankheit wiederholt.

7. Anzeigesteuervorrichtung nach einem der Ansprüche 1 bis 5, derart ausgebildet, dass, wenn mehrere Krankheiten für den ersten Patienten diagnostiziert sind, die Identifikationseinheit (12) für eine überlappungsfreie Zeitspanne Information über die Krankheitsdauer jeder Krankheit des ersten Patienten auf dem Anzeigebildschirm anzeigt, eine Benutzereingabe empfängt, die eine Zeitspanne basierend auf der angezeigten Information spezifiziert, und die spezifizierte Zeitspanne als die überlappungsfreie Zeitspanne der Zielkrankheit identifiziert.

8. Anzeigesteuervorrichtung nach einem der Ansprüche 1 bis 5, derart ausgebildet, dass, wenn mehrere Krankheiten bei dem ersten Patienten diagnostiziert sind, die Identifikationseinheit (12) für eine überlappungsfreie Zeitspanne eine überlappungsfreie Zeitspanne für jede Krankheit gewinnt unter Verwendung der Information über die Krankheitsdauer jeder Krankheit des ersten Patienten, auswählbar die Information der überlappungsfreien Zeitspanne, die für jede Krankheit erhalten wurde, auf dem Anzeigebildschirm anzeigt, eine Benutzereingabe empfängt, die eine der angezeigten überlappungsfreien Zeitspanne auswählt, und die ausgewählte überlappungsfreie Zeitspanne als die überlappungsfreie Zeitspanne identifiziert.

9. Anzeigesteuerverfahren, das mit Hilfe einer Anzeigesteuervorrichtung für medizinische Pflegeinformation ausgeführt wird, welche Vorrichtung mit einer Informationsgewinnungseinheit (11), einer Identifikationseinheit (12) für eine überlappungsfreie Zeitspanne, einer Erzeugungseinheit (13) für eine Anzeigepostenmenge, und eine Anzeigesteuereinheit (14) ausgestattet ist, wobei:
die Informationsgewinnungseinheit (11) Information über medizinische Pflegedaten eines ersten Patienten entsprechend jedem von einer Mehrzahl medizinischer Pflegeposten, Information über Registrierungsdatum und -zeit der medizinischen Pflegedaten und Information über Krankheitsdauer vom Start bis zum Ende jeder Krankheit des Patienten gewinnt;
die Identifikationseinheit (12) für die überlappungsfreie Zeitspanne unter Verwendung von Information über Krankheitsdauer jeder Krankheit eines ersten Patienten, die von der Informationsgewinnungseinheit (11) gewonnen wurde, eine überlappungsfreie Zeitspanne der Krankheitsdauer einer Zielkrankheit identifiziert, bei der es sich um eine der Krankheiten des ersten Patienten handelt, welche überlappungsfreie Zeitspanne sich nicht überlappt mit Krankheitsdauern der übrigen Krankheiten;
die Erzeugungseinheit (13) für eine Anzeigepostenmenge unter Verwendung von medizinischen Pflegedaten des ersten Patienten und Information von Registrierungsdatum und -zeit der medizinischen Pflegedaten, die von der Informationsgewinnungseinheit (11) gewonnen wurde, aus den medizinischen Pflegedaten des ersten Patienten medizinische Pflegedaten extrahiert, deren Registrierungsdatum und -zeit in der überlappungsfreien Zeitspanne der Zielkrankheit enthalten ist, und eine Anzeigepostenmenge erzeugt, zusammengesetzt aus dem medizinischen Pflegeposten entsprechend den extrahierten medizinischen Pflegedaten;
die Informationsgewinnungseinheit (11) Information über medizinische Pflegedaten eines zweiten Patienten gewinnt, der der Zielkrankheit entsprechend jedem von mehreren medizinischen Pflegeposten verdächtig ist; und
die Anzeigesteuereinheit (14) unter Verwendung der Information über medizinische Pflegedaten des zweiten Patienten, die von der Informationsgewinnungseinheit (11) erhalten wurden, auf einem Anzeigebildschirm die medizinischen Pflegedaten von denjenigen des zweiten Patienten anzeigt, die dem medizinischen Pflegeposten der Anzeigepostenmenge entspricht, die von der Erzeugungseinheit (13) für die Anzeigepostenmenge erzeugt wurde.

10. Medium, auf dem ein Anzeigesteuerprogramm für medizinische Pflegeinformation aufgezeichnet ist, welches einen Computer veranlasst zu fungieren als:
eine Informationsgewinnungseinheit (11), ausgebildet zum Gewinnen von Information medizinischer Pflegedaten eines Patienten, entsprechend jedem von mehreren medizinischen Pflegeposten, Information von Registrierungsdatum und - zeit der medizinischen Pflegedaten, und Information über Krankheitsdauer von dem Start bis zum Ende jeder Krankheit des Patienten;
eine Identifikationseinheit (12) für eine überlappungsfreie Zeitspanne, ausgebildet, um anhand von Information über Krankheitsdauer jeder Krankheit eines ersten Patienten, gewonnen von der Informationsgewinnungseinheit (11), eine überlappungsfreie Zeitspanne für Krankheitsdauer einer Zielkrankheit zu identifizieren, bei der es sich um eine von den Krankheiten des ersten Patienten handelt, die sich nicht mit Krankheitsdauern anderer Krankheiten überlappt;
eine Erzeugungseinheit (13) für eine Anzeigepostenmenge, ausgebildet, um unter Verwendung von medizinischen Pflegedaten des ersten Patienten und Information von Registrierungsdatum und -zeit der medizinischen Pflegedaten, die von der Informationsgewinnungseinheit (11) erhalten wurden, aus den medizinischen Pflegedaten des ersten Patienten medizinische Pflegedaten zu extrahieren, deren Registrierungsdatum und -zeit in der überlappungsfreien Zeitspanne enthalten ist, und eine Anzeigepostenmenge zu erzeugen, zusammengesetzt aus dem medizinischen Pflegeposten entsprechend den extrahierten medizinischen Pflegedaten; und
eine Anzeigesteuereinheit (14), ausgebildet, um unter Verwendung von Information der medizinischen Pflegedaten eines zweiten Patienten, der der von der Informationsgewinnungseinheit (11) erhaltenen Zielkrankheit verdächtig ist, die medizinischen Pflegedaten von denen des zweiten Patienten entsprechend dem medizinischen Pflegeposten der Anzeigepostenmenge, die von der Erzeugungseinheit (13) für Anzeigepostenmengen erzeugt wurde, auf einem Anzeigebildschirm anzuzeigen.

## Revendications

1. Appareil de commande d'affichage pour informations de soins médicaux (1), comprenant :
une unité d'obtention d'informations (11) apte à obtenir des informations de données de soins médicaux d'un patient correspondant à chacun d'une pluralité d'éléments de soins médicaux, des informations de date et heure d'enregistrement des données de soins médicaux, et des informations de durée de maladie du début à la fin de chaque maladie du patient ;
une unité d'identification de période sans chevauchement (12) apte à identifier, à l'aide d'informations de durée de maladie de chaque maladie d'un premier patient obtenues par l'unité d'obtention d'informations (11), une période sans chevauchement de durée de maladie d'une maladie cible, laquelle est une des maladies du premier patient, laquelle ne présente pas de chevauchement avec des durées de maladie des autres maladies ;
une unité de génération de jeu d'éléments d'affichage (13) apte à extraire, à l'aide de données de soins médicaux du premier patient et d'informations de date et heure d'enregistrement des données de soins médicaux obtenues par l'unité d'obtention d'informations (11), des données de soins médicaux dont les date et heure d'enregistrement sont incluses dans la période sans chevauchement de la maladie cible dans les données de soins médicaux du premier patient, et à générer un jeu d'éléments d'affichage composé de l'élément de soins médicaux correspondant aux données de soins médicaux extraites, et
une unité de commande d'affichage (14) apte à afficher, à l'aide d'informations de données de soins médicaux d'un second patient avec suspicion de la maladie cible obtenue par l'unité d'obtention d'informations (11), les données de soins médicaux parmi celles du second patient correspondant à l'élément de soins médicaux du jeu d'éléments d'affichage généré par l'unité de génération de jeu d'éléments d'affichage (13) sur un écran d'affichage.

2. Appareil de commande d'affichage pour informations de soins médicaux selon la revendication 1, adapté de telle sorte que si des données de soins médicaux du premier patient sont obtenues de manière supplémentaire par l'unité d'obtention d'informations (11) après génération du jeu d'éléments d'affichage et si un élément de soins médicaux est nouvellement ajouté à l'élément de soins médicaux correspondant aux données de soins médicaux dont les date et heure d'enregistrement sont incluses dans la période sans chevauchement, l'unité de génération de jeu d'éléments d'affichage (13) met à jour le jeu d'éléments d'affichage généré en incluant en outre l'élément de soins médicaux ajouté.

3. Appareil de commande d'affichage pour informations de soins médicaux selon la revendication 1 ou 2, adapté de telle sorte que :
l'unité de commande d'affichage (14) présente une fonction pour réaliser, en réaction à une entrée d'utilisateur pour réaliser un affichage mis en valeur ou détaillé en spécifiant un des éléments de soins médicaux sur l'écran d'affichage, où les données de soins médicaux du second patient correspondant à l'élément de soins médicaux du jeu d'éléments d'affichage est affiché, un affichage mis en valeur ou un affichage détaillé de données de soins médicaux correspondant à l'élément de soins médicaux spécifié, et
l'unité de génération de jeu d'éléments d'affichage (13) présente une fonction pour compter un nombre de fois de l'entrée d'utilisateur et pour générer un jeu d'éléments d'affichage composé uniquement d'un élément de soins médicaux parmi ceux du jeu d'éléments d'affichage dont le nombre de fois compté est supérieur ou égal à une valeur seuil prédéterminée.

4. Appareil de commande d'affichage pour informations de soins médicaux selon la revendication 1 ou 2, adapté de telle sorte que :
l'unité de commande d'affichage (14) présente une fonction pour réaliser, en réaction à une entrée d'utilisateur pour réaliser un affichage mis en valeur ou détaillé en spécifiant un des éléments de soins médicaux sur l'écran d'affichage, où les données de soins médicaux du second patient correspondant à l'élément de soins médicaux du jeu d'éléments d'affichage sont affichées, un affichage mis en valeur ou un affichage détaillé de données de soins médicaux correspondant à l'élément de soins médicaux spécifié, et
l'unité de génération de jeu d'éléments d'affichage (13) présente une fonction pour compter un nombre de fois de l'entrée d'utilisateur, et pour générer un jeu d'éléments d'affichage composé uniquement d'un nombre d'éléments de soins médicaux parmi ceux du jeu d'éléments d'affichage, lequel est inférieur ou égal à une valeur seuil prédéterminée dans un ordre décroissant du nombre de fois compté.

5. Appareil de commande d'affichage pour informations de soins médicaux selon l'une quelconque des revendications 1 à 4, adapté de telle sorte que si une pluralité de maladies est diagnostiquée pour le premier patient, l'unité d'identification de période sans chevauchement (12) règle l'une quelconque de la pluralité de maladies comme la maladie cible, et réalise l'identification de la période sans chevauchement de la maladie cible.

6. Appareil de commande d'affichage pour informations de soins médicaux selon l'une quelconque des revendications 1 à 4, adapté de telle sorte que si : une pluralité de maladies est diagnostiquée pour le premier patient,
l'unité d'identification de période sans chevauchement (12) règle chacune de la pluralité de maladies comme la maladie cible dans cet ordre, et répète l'identification de la période sans chevauchement de la maladie cible, et
l'unité de génération de jeu d'éléments d'affichage (13) répète, par rapport à chaque maladie cible, la génération du jeu d'éléments d'affichage à l'aide des informations de la période sans chevauchement de chaque maladie cible.

7. Appareil de commande d'affichage pour informations de soins médicaux selon l'une quelconque des revendications 1 à 5, adapté de telle sorte que si : une pluralité de maladies est diagnostiquée pour le premier patient, l'unité d'identification de période sans chevauchement (12) affiche des informations de la durée de maladie de chaque maladie du premier patient sur l'écran d'affichage, reçoit une entrée d'utilisateur spécifiant une période sur la base des informations affichées, et identifie la période spécifiée comme période sans chevauchement de la maladie cible.

8. Appareil de commande d'affichage pour informations de soins médicaux selon l'une quelconque des revendications 1 à 5, adapté de telle sorte que si une pluralité de maladies est diagnostiquée pour le premier patient, l'unité d'identification de période sans chevauchement (12) obtient une période sans chevauchement de chaque maladie à l'aide des informations de la durée de maladie de chaque maladie du premier patient, affiche d'une manière pouvant être sélectionnée les informations de la période sans chevauchement obtenues pour chaque maladie sur l'écran d'affichage, reçoit une entrée d'utilisateur sélectionnant l'une quelconque des périodes parmi les périodes sans chevauchement affichées, et identifie la période sans chevauchement sélectionnée comme la période sans chevauchement.

9. Procédé de commande d'affichage pour informations de soins médicaux réalisé avec un appareil de commande d'affichage pour informations de soins médicaux doté d'une unité d'obtention d'informations (11), d'une unité d'identification de période sans chevauchement (12), d'une unité de génération de jeu d'éléments d'affichage (13), et d'une unité de commande d'affichage (14), dans lequel :
l'unité d'obtention d'informations (11) obtient des informations de données de soins médicaux d'un premier patient correspondant à chacun d'une pluralité d'éléments de soins médicaux, des informations de date et heure d'enregistrement des données de soins médicaux, et des informations de durée de maladie du début à la fin de chaque maladie du patient ;
l'unité d'identification de période sans chevauchement (12) identifie, à l'aide d'informations de durée de maladie de chaque maladie d'un premier patient obtenues par l'unité d'obtention d'informations (11), une période sans chevauchement de durée de maladie d'une maladie cible, laquelle est une des maladies du premier patient, laquelle ne présente pas de chevauchement avec des durées de maladie des autres maladies ;
l'unité de génération de jeu d'éléments d'affichage (13) extrait, à l'aide de données de soins médicaux du premier patient et d'informations des date et heure d'enregistrement des données de soins médicaux obtenues par l'unité d'obtention d'informations (11), des données de soins médicaux dont les date et heure d'enregistrement sont incluses dans la période sans chevauchement de la maladie cible dans les données de soins médicaux du premier patient, et génère un jeu d'éléments d'affichage composé de l'élément de soins médicaux correspondant aux données de soins médicaux extraites ;
l'unité d'obtention d'informations (11) obtient des informations de données de soins médicaux d'un second patient avec suspicion de la maladie cible correspondant à chaque élément d'une pluralité d'éléments de soins médicaux, et
l'unité de commande d'affichage (14) affiche, à l'aide des informations de données de soins médicaux du second patient obtenues par l'unité d'obtention d'informations (11), les données de soins médicaux parmi celles du second patient correspondant à l'élément de soins médicaux du jeu d'éléments d'affichage généré par l'unité de génération de jeu d'éléments d'affichage (13) sur un écran d'affichage.

10. Support sur lequel est enregistré un programme de commande d'affichage pour informations de soins médicaux pour faire en sorte qu'un ordinateur fonctionne comme :
une unité d'obtention d'informations (11) apte à obtenir des informations de données de soins médicaux d'un patient correspondant à chacun d'une pluralité d'éléments de soins médicaux, des informations de date et heure d'enregistrement des données de soins médicaux, et des informations de durée de maladie du début à la fin de chaque maladie du patient ;
une unité d'identification de période sans chevauchement (12) apte à identifier, à l'aide d'informations de durée de maladie de chaque maladie d'un premier patient obtenues par l'unité d'obtention d'informations (11), une période sans chevauchement de durée de maladie d'une maladie cible, laquelle est une des maladies du premier patient, laquelle ne présente pas de chevauchement avec des durées de maladie des autres maladies ;
une unité de génération de jeu d'éléments d'affichage (13) apte à extraire, à l'aide de données de soins médicaux du premier patient et d'informations de date et heure d'enregistrement des données de soins médicaux obtenues par l'unité d'obtention d'informations (11), des données de soins médicaux dont les date et heure d'enregistrement sont incluses dans la période sans chevauchement de la maladie cible dans les données de soins médicaux du premier patient, et à générer un jeu d'éléments d'affichage composé de l'élément de soins médicaux correspondant aux données de soins médicaux extraites, et
une unité de commande d'affichage (14) apte à afficher, à l'aide d'informations de données de soins médicaux d'un second patient avec suspicion de la maladie cible obtenue par l'unité d'obtention d'informations (11), les données de soins médicaux parmi celles du second patient correspondant à l'élément de soins médicaux du jeu d'éléments d'affichage généré par l'unité de génération de jeu d'éléments d'affichage (13) sur un écran d'affichage.
